# EUROPEAN PATENT SPECIFICATION

(11) **EP 4 659 723 B1**
(45) Date of publication and mention of the grant of the patent: **23.09.2026**
(21) Application number: 24180260.2
(22) Date of filing: 05.06.2024
(51) Int. Cl.: A61F 13/15

(54) **AN APPARATUS AND A METHOD FOR STRETCHING AN ELASTIC STRIP IN A CROSS-MACHINE DIRECTION**
VORRICHTUNG UND VERFAHREN ZUM STRECKEN EINES ELASTISCHEN BANDES IN MASCHINENÜBERGREIFENDER RICHTUNG
APPAREIL ET PROCÉDÉ POUR ÉTIRER UNE BANDE ÉLASTIQUE DANS UNE DIRECTION TRANSVERSALE À LA MACHINE

(43) Date of publication of application: 10.12.2025
(73) Proprietor: Curt G. Joa, Inc., Sheboygan Falls, Wisconsin 53085-0903 (US)
(72) Inventor: Giffey, Zachary J, Sheboygan Falls, 53085-0903 (US); Lawrence, Cristopher S, Sheboygan Falls, 53085-0903 (US)
(74) Representative: Potter Clarkson

(56) References cited:
- US-A1- 2012 073 729

## Description

The present invention relates to an apparatus and a method for stretching at least one elastic strip in a cross-machine direction. The present invention also relates to the use of such an apparatus or a method to assemble an elastic laminate and/or to manufacture an absorbent article, such as a diaper or incontinence control garment. The following prior art document was cited in the European Search Report: US 2012073729 A1.

Absorbent articles, such as infant diapers, incontinence garments and the like, are typically formed from a combination of multiple web layers, absorbent structures, and elastic elements. The absorbent articles may include a thin flexible liquid impermeable backing sheet on which a permeable nonwoven sheet is overlayed. An absorbent pad is disposed between the two layers and the layers are adhered at their edges to form a unitary article that prevents liquid body exudates from seeping out of the edges of the absorbent article. Elastic elements, such as an elastic waist element or pockets, elastic ear panels, and elastic leg cuffs, may be provided in the absorbent article to help the absorbent article better conform to the body of the wearer around the waist, back, and legs to prevent leakage along the interface of the nonwoven layer which is in contact with the body. Elastic elements may also be provided in disposable medical products, for example as wrist cuffs in medical gowns.

In the manufacture of absorbent articles, elastic elements are often provided in the form of an arrangement of elastic strands (i.e. single thread-like lengths of elastic material used to impart linear elasticity) or elastic strips (i.e. broader and flatter ribbon- or sheet-like lengths of elastic material capable of stretching in one or in multiple directions for imparting elasticity over a larger area) which may be provided adjacent a single nonwoven sheet or sandwiched between a top sheet and a back sheet, and which may be bonded to secure them in place.

Elastic strips may be applied to a web layer in a stretched condition. Some conventional methods for stretching elastic strips utilize a spreader mechanism comprising a pair of canted discs to stretch an elastic strip in the cross-machine direction before it is applied to a web layer.

The spreader mechanism comprises a first rotatable disc and a second rotatable disc having respective first and second axes of rotation inclined with respect to each other. An elastic strip is fed towards and guided over the outer rims of the first and the second rotatable discs. Since the first and the second rotatable discs are positioned at an angle to the direction of movement of the elastic film, the angled orientation causes a pulling or stretching force on the elastic strip as it passes over the first and the second rotatable discs during operation, which causes the elastic strip to stretch in the cross-machine direction.

The outer rims of the canted discs may comprise apertures that are fluidly connected to a vacuum pressure source so that vacuum air pressure may be used to hold the edges of the elastic strip on the outer rims of the discs during operation.

Splicing tape, which is often used to attach an already machine-threaded elastic strip to a new elastic strip roll, can however cause the canted discs of a spreader mechanism to lose their grip on the edges of an elastic strip when a splice section passes over the canted discs. This can cause a length of elastic strip located downstream of the splicing tape in the machine direction to contract to a narrower width than desired prior to lamination, which may lead to the production of an unusable final product. Additionally, the edges of an elastic strip downstream of the splicing tape, may require manual repositioning onto the canted discs of the spreader mechanism, which extends the downtime, resulting in delays and interruptions in production.

In an aspect of the invention there is provided an apparatus for stretching an elastic strip in a cross-machine direction comprising the features recited in claim 1.

The apparatus comprises a set of rotatable discs including a first rotatable disc and a second rotatable disc. The first rotatable disc is inclined at an angle with respect to the second rotatable disc. Each rotatable disc has an outer surface comprising at least one aperture that is configured to be fluidly connected to a vacuum pressure source and that is configured to receive an edge of an elastic strip at a pick-up location and hold the edge of the elastic strip as the elastic strip is advanced in the machine direction from the pick-up location to a drop-off location during operation, whereby the distance between the first rotatable disc and the second rotatable disc is greater at the drop-off location than at the pick-up location, thereby stretching the elastic strip in the cross-machine direction. The outer surface of each rotatable disc also comprises at least one row of teeth, that extends around at least part of a periphery of the rotatable disc and that comprises a plurality of teeth each having a gripping end, whereby each tooth of the plurality of teeth exhibits a maximum cross-section that is situated at a distance from at the gripping end of the tooth.

The at least one aperture, which may be formed within the outer surface or recessed below and configured to be fluidly coupled thereto, retains the elastic strip on the rotatable discs between the pick-up and drop-off locations as the elastic strip is advanced in the machine direction from the pick-up location to the drop-off location during operation. The at least one row of teeth helps to retain the cross-machine-stretch of the elastic strip as a splice section passes over the rotatable discs. This maintains consistent tension across the elastic strip and prevents the elastic strip from contracting to a narrower width than desired prior to lamination, which minimizes waste and enhances the quality of end products. The at least one row of teeth prevents slippage of an elastic strip in both the cross-machine direction and the machine direction, which reduces or eliminates the need for manual repositioning of the edges of an elastic strip onto the rotatable discs after a splice section has passed over the rotatable discs, which reduces the downtime, and consequently delays and interruptions in production.

The gripping ends of the teeth engage an edge of an elastic strip that is pulled toward the at least one aperture by the vacuum pressure source during operation. The teeth on the rotatable discs pull the the edges of the elastic strip in the axial direction and machine direction, which maintains the elastic strip in position in a stretched condition on the rotatable discs and also encourages the elastic strip to rethread (i.e., to re-stretch onto the rotatable discs in the axial direction following a splice). The at least one row of teeth of the outer surface of each rotatable disc thereby helps to maintain the hold on the edges of the elastic strip as the elastic strip is advanced in the machine direction from the pick-up location to the drop-off location during operation.

According to an embodiment, the outer surface of a rotational disc may comprise at least two rows of teeth. Additional rows of teeth, such as a second row of teeth, may provide additional pull on the elastic strip in the axial direction and may further aid in rethreading the elastic strip following a splice. The edge of an elastic strip may be pulled between rows of teeth by the vacuum pressure source.

According to an embodiment, the outer surface comprises at least one groove adjacent to the at least one row of teeth, i.e. the outer surface may comprise a groove located next to one row of teeth or next to each row of teeth, or a groove located between rows of teeth. The groove may extend radially inward from the outer surface. The groove may extend around at least part of the circumference of a rotatable disc. The at least one groove aids the gripping ends of the teeth in engaging an edge of an elastic strip and pulling the the edge of the elastic strip in the axial direction and machine direction, which aids in maintaining the elastic strip in position on a rotatable disc in a stretched condition and also aids in encouraging the elastic strip to rethread.

According to an embodiment, the at least one row of teeth lies flush with the outer surface, of a rotatable disc, i.e. the at least one row of teeth does not protrude radially outwards from the outer surface of a rotatable disc.

Alternatively, the at least one row s of teeth protrudes radially outwards from the outer surface of the rotatable disc.

According to an embodiment, some teeth or all of the teeth, of the at least one row of teeth comprise(s) a pointed end, a rounded end, or a flat end or a rectangular end, whereby an end of a tooth is a part of a tooth that is arranged to come into contact with an edge of an elastic strip. A tooth may be V-shaped, U-shaped, or substantially bar-shaped. The plurality of teeth may have a pointed, flat, or rounded gripping end.

According to an embodiment, the outer surface of at least one rotatable disc comprises a plurality of apertures, at least one row of apertures, or a pattern of apertures, that extends around at least part of the periphery of the at least one rotatable disc and that is configured to be fluidly connected to the vacuum pressure source.

A rotatable disc according to any of the embodiments described herein may have an outer surface comprising at least one aperture of any suitable size. The outer surface may comprise any number of apertures, and/or any number of rows of apertures arranged in any suitable manner. The size and/or number and/or arrangement of apertures may be determined as a function of the breathability of the elastic strip that is to be stretched in a cross-machine direction. A more breathable elastic strip requires more vacuum to retain the elastic strip on the outer surface of a rotatable disc.

According to an embodiment, the outer surface of at least one rotatable disc is integrally formed with the at least one row of teeth. An outer surface of a rotatable disc and the at least one row of teeth may for example be machined from a single piece of a material, such as a continuous block of material. This increases a manufacturer's design options and facilitates the creation of at least one custom-made row of teeth. Teeth that apply varying degrees of stretch to an elastic strip can thereby be paired with different elastic strips.

Alternatively, the outer surface of at least one rotatable disc may be configured to receive at least one insert including at least one row of teeth to provide the at least one row of teeth. Different inserts may thereby be used to enable an operator to modify the arrangement of the at least one row of teeth on the outer surface of a rotatable disc and/or the tooth design used for a particular elastic strip.

According to an embodiment, the first rotatable disc of the set of rotatable discs is configured to rotate about a first axis of rotation, and a second rotatable disc of the set of rotatable discs is configured to rotate about a second axis of rotation, whereby the first axis of rotation is inclined at an angle with respect to the second axis of rotation.

Alternatively, the first rotatable disc of the set of rotatable discs and the second rotatable disc of the set of rotatable discs are configured to rotate about a common axis of rotation.

According to an embodiment, the apparatus comprises at least one additional set of rotatable discs for stretching at least one additional elastic strip in a cross-machine direction. An apparatus may comprise any number of sets of rotatable discs located in any desired arrangement along the cross-machine direction and/or the machine direction, such as at least two, at least three, at least four, at least five, at least six, at least seven, at least eight, at least nine, at least ten sets of rotatable discs. An apparatus may comprise at least one, or a plurality of pairs of rotatable discs.

According to an embodiment, the apparatus comprises at least one supply unit configured to supply an elastic strip to the set of rotatable discs, at least one supply unit configured to supply at least one carrier web, and at least one bonding device configured to bond the stretched elastic strip to the at least one carrier web. A carrier web may be a nonwoven layer or any other component of an elastic laminate or absorbent article.

The at least one bonding device may comprise at least one or a combination of the following: an adhesive bonding device, an ultrasonic bonding device, a heat bonding device, a pressure bonding device, a heat and pressure bonding device. At least one bonding device may be located at any one or more of the following positions: upstream of the pick-up location of a set of rotatable discs in the machine direction, at the pick-up location of a set of rotatable discs, downstream of the pick-up location of a set of rotatable discs in the machine direction, at the drop-off location of a set of rotatable discs, downstream of the drop-off location of a set of rotatable discs in the machine direction.

According to an embodiment, the apparatus comprises a support structure and at least one of the rotatable discs is mounted on the support structure. At least one rotatable disc may be slidably mounted on the support structure. Alternatively, or additionally, at least one rotatable disc may be fixedly mounted on the support structure.

A first rotatable disc of a set of rotatable discs, may be mounted in a fixed position relative to a second rotatable disc of the set of rotatable discs, whereby the spacing between the first rotatable disc and the second rotatable disc is fixed and/or the angular relationship between the first rotatable disc and the second rotatable disc is fixed. In embodiments of the apparatus comprising a plurality of sets of rotatable discs, at least one set of rotatable discs may be mounted in a fixed position relative to one or more other sets of rotatable discs.

One or more of the following parameters may be adjusted by moving the at least one rotatable disc on the support structure: the minimum distance and/or the maximum distance, a spacing between a first set of rotatable discs and a second set or rotatable discs, and/or the inclination of the first rotatable disc or the first axis of rotation and/or the inclination of the second rotatable disc or the second axis of rotation.

Such a support structure enables the pitch of an elastic strip, i.e. the distance between adjacent points in a pattern of elastic, which is typically measured along the length of the elastic strip, and/or the spacing between adjacent elastic strips applied onto a carrier web to be varied as desired.

According to an embodiment, the support structure is a common support structure, and all of the rotatable discs are mounted on the common support structure.

According to an embodiment, the support structure or common support structure, comprises at least one rotatable disc-position indicator, such as a marking, a ruler and/or a protractor, configured to indicate a position of the at least one rotatable disc that is mounted thereon.

In another aspect of the invention there is provided a method for stretching an elastic strip in a cross-machine direction.

The method comprises the features recited in the independent method claim.

The method comprises supplying an elastic strip to a set of rotatable discs including a first rotatable disc and a second rotatable disc, whereby each rotatable disc has an outer surface comprising at least one aperture that is configured to be fluidly connected to a vacuum pressure source. The method comprises rotating the first rotatable disc and the second rotatable disc, whereby the first rotatable disc is inclined at an angle with respect to the second rotatable disc. The method also comprises receiving an edge of an elastic strip on the outer surface of the first rotatable disc and an opposing edge of the elastic strip on an outer surface of the second rotatable disc at a pick-up location, wherein the outer surface of each rotatable disc comprises at least one row of teeth that extends around at least part of a periphery of a rotatable disc, and the at least one row of teeth comprises a plurality of teeth each having a gripping end, whereby each tooth of the plurality of teeth exhibits a maximum cross-section that is situated at a distance from at the gripping end of the tooth. The method comprises holding an edge of the elastic strip on the outer surface of each rotatable disc using the vacuum pressure source and advancing the elastic strip in the machine direction from the pick-up location to a drop-off location, whereby the distance between the first rotatable disc and the second rotatable disc is greater at the drop-off location than at the pick-up location, thereby stretching the elastic strip in the cross-machine direction.

According to an embodiment, the outer surface comprises at least one groove adjacent to the at least one row of teeth,

According to an embodiment, the outer surface of each rotatable disc of a set of rotatable discs comprises at least one row of teeth that lies flush with the outer surface of a rotatable disc.

According to an embodiment, the outer surface of each rotatable disc of a set of rotatable discs comprises at least one row of teeth that protrudes radially outwards from the outer surface of a rotatable disc.

According to an embodiment, the outer surface is integrally formed with the at least one row of teeth.

According to an embodiment, the outer surface of at least one rotatable disc comprises a plurality of apertures, at least one row of apertures. Or a pattern of apertures that extends around at least part of a periphery of a rotatable disc, whereby the apertures are fluidly connected to the vacuum pressure source.

According to an embodiment, the method comprises adjusting an intensity of the vacuum pressure applied on the edges of the elastic strip during operation, such as by selectively operating a vacuum pump that provides a vacuum within the rotatable discs.

According to an embodiment, the method comprises rotating the first rotatable disc of the set of rotatable discs about a first axis of rotation, and the second rotatable disc of the set of rotatable discs about a second axis of rotation, whereby the first axis of rotation is inclined at an angle with respect to the second axis of rotation.

Alternatively, the method comprises rotating the first rotatable disc of the set of rotatable discs and the second rotatable disc of the set of rotatable discs about a common axis of rotation.

According to an embodiment, the method comprises supplying at least one carrier web, and bonding at least one stretched elastic strip to the at least one carrier web.

According to an embodiment, the method comprises supplying two carrier webs and bonding at least one stretched elastic strip between the two carrier webs.

According to an embodiment, the method comprises mounting at least one rotatable disc on a support structure. The method may comprise slidably mounting or fixedly mounting at least one rotatable disc on the support structure. At least one of the following parameters may be adjusted by moving the at least one rotatable disc on the support structure: the minimum distance and/or the maximum distance, a spacing between a first set of rotatable discs and a second set of rotatable discs, and/or the inclination of the first rotatable disc or the first axis of rotation and/or the inclination of the second rotatable disc or the second axis of rotation.

According to an embodiment, the method comprises providing a common support structure and mounting all of the rotatable discs on the common support structure.

According to an embodiment, the method comprises providing the support structure, or the common support structure, with at least one rotatable disc-position indicator to indicate a position of the at least one rotatable disc mounted on the support structure.

A system according to any of the embodiments of the invention may be used to carry out a method according to any of the embodiments of the invention. A method according to any of the embodiments of the invention may be carried out using a system according to any of the embodiments of the invention.

In a further aspect of the invention there is provided the use of an apparatus according to any of the embodiments described herein, or a method according to any of the embodiments described herein to assemble an elastic laminate and/or to manufacture an absorbent article, such as a disposable absorbent article.

All embodiments of the invention and particular features mentioned herein may be taken in isolation or in combination with any other embodiments and/or particular features mentioned herein (hence describing more particular embodiments and particular features as disclosed herein) without departing from the disclosure of the invention.

Unless otherwise indicated, all technical and scientific terms used herein have the same meaning as commonly understood by one of ordinary skill in the art to which this invention pertains.

As used herein, the term "elastic" is intended to mean the ability of a material to stretch by at least 5%, or at least 10%, or at least 20%, or at least 30%, or at least 40%, or at least 50% of its original length without rupture and return to its original size and shape.

The term "elastic strip" is intended to mean a means a ribbon- or sheet-like material wherein the length and width of the material far exceed the thickness of the material. For example, the length and width of the material are each at least 10 times, or at least 20 times, or at least 30 times, or at least 40 times, or at least 50 times, or at least 100 times, or at least 200 times, or at least 500 times its thickness. An elastic strip may have a width of at least 1 cm, or at least 2 cm, or at least 3 cm, or at least 4 cm, or at least 5 cm, or at least 6 cm, or at least 7 cm, or at least 8 cm, or at least 9 cm, or at least 10m measured in a cross-machine direction in a non-stretched condition. The term "elastic strip" does not include elastic strands, i.e. single thread-like lengths of elastic material used to impart linear elasticity, which have a width of less than 1 cm measured in a cross-machine direction in a non-stretched condition. An elastic strip may be supplied in the form of a continuous elastic strip, or a discontinuous elastic strip, i.e. an elastic strip supplied as a series of discrete elastic strips.

The term "an edge of an elastic strip" is intended to mean not only an outer boundary of the elastic strip in a stretched or unstretched condition in a cross-machine direction, but also an area of the stretched or unstretched elastic strip within a distance from the outer boundary of the stretched or unstretched elastic strip in a cross-machine direction. The distance may correspond to up to 30%, or up to 20%, or up to 10%, or up to 5% of the total extension of the stretched or unstretched elastic strip in the cross-machine direction.

The term "rotatable disc" is intended to mean any mechanical component that is capable of turning or rotating around its axis of rotation. A rotatable disc may be of any suitable shape and/or of any suitable size and not necessarily a circular object. A rotatable disc may comprise a circular disc, a conical disc, a ring, a segmented ring comprising a plurality of ring segments arranged around a central axis, a portion of a sphere. A rotatable disc may have a regular or an irregular shape.

Likewise, terms such as "periphery", "radial direction" and "axial direction" are not intended to imply that a rotatable disc is necessarily a circular object, but are intended to also include the corresponding term when a "rotatable disc" is not a circular object, such as "perimeter", "a direction outwards from the rotational axis of the "rotatable discʺʺ and "a direction parallel to the axis of rotation of the "rotatable discʺʺ respectively.

A "set" of rotatable discs may consist of, or comprise a pair of rotatable discs. The rotatable discs may be of any size and/or any shape. In embodiments of the apparatus and method comprising a plurality of sets of rotatable discs, the rotatable discs of a first set of rotatable discs may have a different size and/or a different shape than the rotatable discs of one or more other sets of rotatable discs.

The term "machine direction" (MD) is used herein to refer to the direction of material flow through a process.

The term "cross-machine direction" (CD) is used herein to refer to a direction that is perpendicular, or substantially perpendicular, to the machine direction.

The term "minimum distance" is intended to mean the smallest length of space between the first rotatable disc and the second rotatable disc of a set of rotatable discs in the cross-machine direction measured between the innermost parts of the outer surfaces of the set of rotatable discs in the cross-machine direction. In the apparatus according to the invention, the edges of an elastic strip may be picked up at the location at which the first rotatable disc and the second rotatable disc of a set of rotatable discs are at a minimum distance from each other, or at a location upstream or downstream from that location. The elastic strip is then advanced in the machine direction towards the location at which the first rotatable disc and the second rotatable disc of a set of rotatable discs are at a maximum distance from each other, or at a location upstream or downstream from that location, thereby stretching the elastic strip in the cross-machine direction. The stretched elastic strip may then be taken off the set of rotatable discs, by transferring the stretched elastic strip to a rotating anvil or to a transfer roll for example.

The term "maximum distance" is intended to mean the greatest length of space between the first rotatable disc and the second rotatable disc of a set of rotatable discs space measured between the innermost parts of the outer surfaces the set of rotatable discs in the cross-machine direction.

The term "teeth" is intended to mean projecting parts on a rotatable disc, which function to engage with an edge of an elastic strip that is pulled toward the at least one aperture in the outer surface of a rotatable disc by the vacuum pressure source and grip it while the elastic strand is advanced from the pick-up location to the drop-off location of a set of rotatable discs in the machine direction during operation.

The "gripping end" of a tooth is the part or parts of a tooth configured to come into contact with an edge of an elastic strip during the operation of the apparatus.

The term "cross-section of a tooth" is the surface that would be exposed by making a straight cut through the tooth in a radial direction of the rotatable disc.

The term "whereby a tooth exhibits a maximum cross-section that is situated at a distance from at the gripping end of the tooth" is intended to mean that the tooth does not have a uniform cross-section but a non-uniform cross-section. The maximum cross-section of the tooth is not situated at the gripping end of the tooth but may be situated at the opposing end of the tooth or between the gripping end and the opposing end of the tooth.

The term "row of teeth" is intended to mean a continuous or non-continuous, straight or curved line or arrangement of teeth. A row of teeth may extend along at least one part of the outer surface of a rotatable disc, or around the entire outer surface of a rotatable disc. A row of teeth may be configured to interlock with an adjacent row of teeth.

According to an embodiment of the invention, the apparatus may comprise equipment to facilitate the release of an edge of a stretched elastic strip from the at least one aperture and the at least one row of teeth at the drop-off location.

The term "disposable absorbent article" is intended to mean an absorbent article that is not intended to be laundered or otherwise restored and re-used as an absorbent article. Instead, a disposable absorbent article is intended to be discarded after a single use and preferably recycled.

The term "comprises" will take its usual meaning in the art, namely indicating that the component includes but is not limited to the relevant features (i.e., including, among other things). As such, the term "comprises" will include references to the component consisting essentially of the relevant feature(s). For the avoidance of doubt, the term "comprises" will also include references to the component "consisting essentially of" (and in particular "consisting of") the relevant feature(s).

The invention is illustrated by way of the following examples, which are not intended to be limiting on the general scope of the invention.
**Figure 1****:** schematically shows a side view of an apparatus according to an embodiment of the invention operating to assemble an elastic laminate,
**Figure 2****:** schematically shows a plan view of part of an apparatus according to an embodiment of the invention,
**Figure 3****:** schematically shows a pair of rotatable discs of an apparatus according to an embodiment of the invention,
**Figure 4****:** schematically shows a plan view of an outer surface of a rotatable disc of an apparatus according to an embodiment of the invention,
**Figures 5A, 5B, 6A & 6B****:** schematically show examples of outwardly protruding teeth and a corresponding row of apertures of a rotatable disc according to embodiments of the invention,
**Figures 7A to 10B****:** schematically show examples of non-outwardly protruding teeth and a corresponding plurality of apertures of a rotatable disc according to embodiments of the invention,
**Figure 11****:** is a perspective view of a support structure an apparatus according to an embodiment of the invention, and
**Figure 12****:** is a flow chart showing the steps of a method according to an embodiment of the invention.

The drawings have not necessarily been drawn to scale and the dimensions of certain features may have been exaggerated for the sake of clarity.

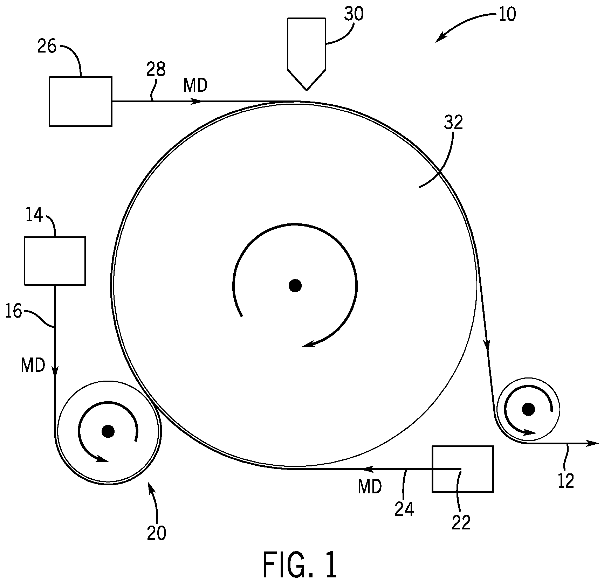

Figure 1 schematically shows a side view of an apparatus 10 according to an embodiment of the invention operating to assemble an elastic laminate 12 comprising an elastic strip 16. The apparatus 10 may be used to manufacture an absorbent article, such as a disposable absorbent article, or to manufacture an intermediary composite material for later use in the manufacture of an absorbent article.

The apparatus 10 comprises a supply unit 22 configured to supply a first carrier web 24, to an outer surface of a rotating anvil 32, i.e. a cylindrical or drum-like component that is configured to rotate continuously during the manufacturing process. The first carrier web 24 may be a liquid-permeable or liquid impermeable nonwoven layer, or any other component of an absorbent article.

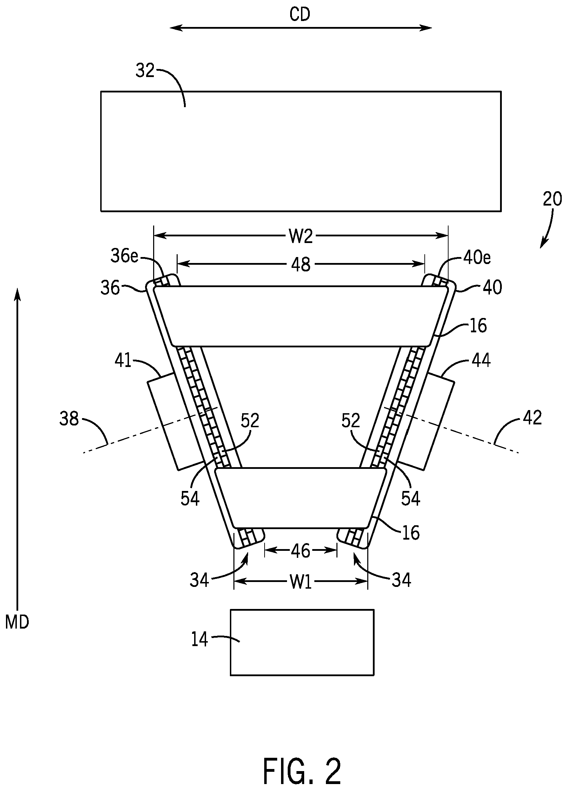

The apparatus 10 also comprises a supply unit 14 configured to supply at least one elastic strip 16 to a spreader mechanism 20 for stretching the elastic strip 16 in a cross-machine direction. The spreader mechanism will be described in more detail with reference to Figure 2.

The apparatus 10 illustrated in Figure 1 further comprises a supply unit 26 configured to supply a second carrier web 28 to the layers on the outer surface of the rotating anvil 32. The second carrier web 28 may be a liquid-permeable or liquid impermeable nonwoven layer, or any other component of an absorbent article.

The apparatus 10 also comprises a bonding device 30, such as an ultrasonic horn, configured to bond the stretched first elastic strip 16 between the first carrier web 24 and the second carrier web 28.

During the operation of the apparatus 10, the first carrier web 24 advances in the machine direction MD towards and along the outer surface of a rotating anvil 32. The spreader mechanism 20 stretches the elastic strip 16 in a cross-machine direction. The stretched elastic strip 16 is then positioned into contact with the first carrier web 24 on the outer surface of a rotating anvil 32 and maintained in a stretched condition. The second carrier web 28 is advanced and positioned into contact with and attached to the layers on the outer surface of the rotating anvil 32.

As the anvil 32 rotates, the first carrier web 24, the stretched elastic strip 16 and the second carrier web 28 are advanced between the outer surface of the anvil 32 and the bonding device 30 to bond the first carrier web 24, the stretched elastic strip 16, and the second carrier web 28 together to form an elastic laminate 12. The elastic laminate 12 may then be advanced from the anvil 32 to one or more additional absorbent article assembly processes or stored until needed for production. In a relaxed state, the stretched elastic strip 16 will contract in the cross-machine direction.

Continuous operation of such an apparatus 10 permits the high-speed assembly of elastic laminates 12 comprising at least one elastic strip 16, which in turn facilitates the high-speed manufacture of absorbent articles, such as diapers or incontinence control garments.

Figure 2 schematically shows a plan view of a set of rotatable discs 34 of a spreader mechanism 20 for stretching an elastic strip 16 in a cross-machine direction CD. A spreader mechanism 20 of an apparatus 10 according to the present invention may comprises one or more sets of such rotatable discs 34 for stretching one or more elastic strips 16 in a cross-machine direction CD.

In the illustrated embodiment, an elastic strip 16 is supplied to the spreader mechanism 20 in the form of a non-continuous elastic strip 16, i.e. in a series of discrete elastic strips 16. Alternatively, the elastic strip 16 is supplied to the spreader mechanism 20 in the form of a continuous elastic strip 16.

According to an embodiment, the apparatus 10 according to the present invention may comprise at least one cutting device, such as at least one rotating or oscillating cutting element (not shown), which is configured to cut at least one continuous elastic strip into a plurality of non-continuous elastic strips.

The spreader mechanism 20 comprises a set of rotatable discs comprising a first rotatable disc 36 that is configured to rotate about a first axis of rotation 38, and a second rotatable disc 40 that is configured to rotate about a second axis of rotation 42. The first rotatable disc 36 may comprise a hub or be mounted on a first shaft 41. Likewise, the second rotatable disc 36 may comprise a hub or be mounted on a second shaft 44. The first axis of rotation 38 is inclined at an angle with respect to the second axis of rotation 42. In an alternative embodiment, first and second discs 36, 40 are oriented such that they are inclined at an angle relative to one another, as shown in Figure 2, but configured to rotate about a common axis of rotation via a constant velocity (CV) drive (not shown).

Each rotatable disc 36, 40 has an outer surface 36e, 40e that is configured to receive an edge of an elastic strip 16 having a width W1 at a pick-up location and hold the edge of the elastic strip 16 as the elastic strip 16 is advanced in the machine direction MD from the pick-up location to a drop-off location during operation, whereby the distance between the first rotatable disc and the second rotatable disc is greater at the drop-off location than at the pick-up location. Each rotatable disc 36, 40 comprises at least one aperture (not shown in Figure 2) that is configured to be fluidly connected to a vacuum pressure source (not shown). The aperture(s) may be formed within the outer surface 36e, 40e or recessed below and fluidly coupled thereto.

The angled orientation of the first rotatable disc 36 and the second rotatable disc 40 creates a pulling or stretching force on the elastic strip 16 whose edges are held at the outer surfaces 36e and 40e of the first rotatable disc 36 and the second rotatable disc 40 by at least one aperture that is configured to be fluidly connected to a vacuum pressure source, thereby stretching the elastic strip 16 to a width W2 in the cross-machine direction CD as the rotatable discs 36, 40 rotate. The stretching of an elastic strip 16 in the cross-machine direction CD may thereby be precisely controlled and the use of such a spreader mechanism 20 in the manufacture of absorbent articles helps to provide the flexibility for the absorbent articles to fit comfortably and effectively, ensuring consistent and reliable performance.

The outer surface 36e, 40e of each rotatable disc 36, 40 also comprises a plurality of rows of teeth 52, 54. In the illustrated embodiment each row of teeth 52, 54 extends around the entire periphery of each rotatable disc 36, 40. The plurality of rows of teeth 52, 54 of each rotatable disc 36, 40 are configured to grip an edge of the elastic strip 16 that is pulled toward the at least one aperture and between the rows of teeth 52, 54 by the vacuum pressure source and maintain the hold on the edge of the elastic strip 16 as the elastic strip 16 is advanced in the machine direction MD from the pick-up location to the drop-off location during operation.

It should be noted that both the first rotatable disc 36 and the second rotatable disc 40 of a set of rotatable discs 34 do not necessarily have to be positioned at an angle to the machine direction MD. According to an embodiment, either the first rotatable disc 36 or the second rotatable disc 44 of a set of rotatable discs 34 may be configured to rotate about an axis of rotation that extends along the cross-machine direction CD as long as the first axis of rotation 38 about which the first rotatable disc 36 rotates is inclined at an angle with respect to the second axis of rotation 42 about which the second rotatable disc 40 rotates.

Furthermore, the pick-up location and the drop-off location need not necessarily correspond to the exact locations where the first rotatable disc 36 and the second rotatable disc 40 of a set of rotatable discs 34 are at a minimum distance 46 and a maximum distance 48 respectively. An elastic strip 16 may be picked up by a set of rotatable discs 34 at any location downstream of the minimum distance 46, and it may be taken off the set of rotatable discs 34 at any location upstream of the maximum distance 48 in the machine direction MD.

A stretched elastic strip 16 may be transferred to the outer surface of a rotating anvil 32 directly, or indirectly via one or more transfer rolls.

The rotatable discs 34, 62 may be configured to rotate at a constant speed, or a non-constant speed.

According to an embodiment, an apparatus according to the invention may comprise at least one optional optical camera (not shown) to monitor the advancement of an elastic strip 16 over the rotatable discs 36, 40. The advancement of a spliced section of elastic strip 16 16 over the rotatable discs 36, 40 may thereby be monitored.

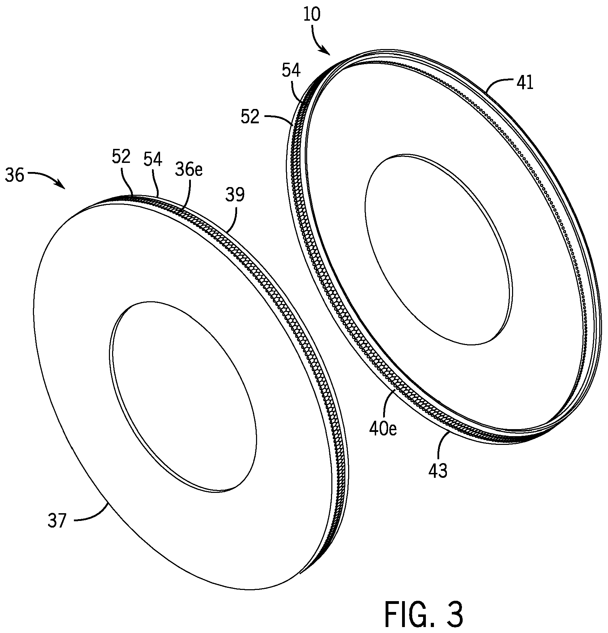

Figure 3 schematically shows rotatable discs 36, 40 of an apparatus according to an embodiment of the invention. The rotatable discs 36, 40 each have an outer surface 36e, 40e comprising two rows of teeth 52, 54. In the illustrated embodiment the rows of teeth 52, 54 extend around the entire periphery of the rotatable discs 36, 40. According to an embodiment, a row of teeth 52, 54 may extend around only one or more parts of the periphery of a rotatable disc 36, 40.

A rotatable disc 36, 40 may comprise a row of teeth 52, at least two rows of teeth 52, 54, or at least three rows of teeth 52, 54, or at least four rows of teeth 52, 54, or at least five rows of teeth 52, 54, or at least six rows of teeth 52, 54, or at least seven rows of teeth 52, 54, or at least eight rows of teeth 52, 54, or at least nine rows of teeth 52, 54, or at least ten rows of teeth 52, 54. According to an embodiment a rotatable disc 36, 40 may comprise at least one pair of rows of teeth 52, 54, at least two pairs of rows of teeth 52, 54, at least three pairs of rows of teeth 52, 54 or at least four pair of rows of teeth 52, 54.

A row of teeth 52 or a plurality of rows of teeth 52, 54 may be machined into an outer surface 36e, 40e of a rotatable disc 36, 40, whereby the outer surface 36e, 40e of a rotatable disc 36, 40 and the at least one row of teeth 52, 54 are integrally formed by a subtractive machining process such as milling, drilling, laser, or electrical discharge machining (EDM), or other known process. Teeth 52, 54 may also be formed via an additive process such as fused deposition modelling (FDM), stereolithography (SLA), or other known process.

In alternative embodiments, rotatable discs 36, 40 may be manufactured as multi-piece assemblies. One or more teeth 52, 54 may be provided as part of a component that is coupled to one or more additional components to form at least part of the outer surface 36e, 40e of a rotatable disc 36, 40, or to form at least part of the rotatable disc 36, 40.

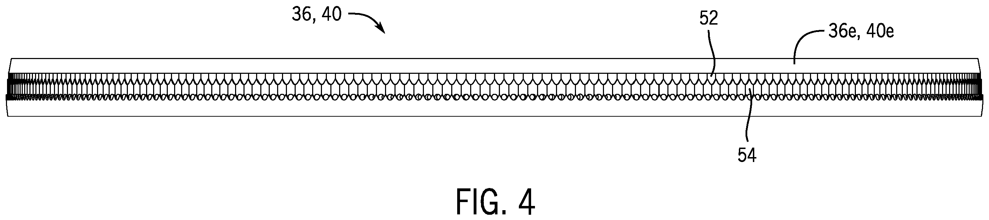

Figure 4 schematically shows a plan view of a rotatable disc 36, 40. A plurality of rows of teeth 52, 54 may be located at the centre of an outer surface 36e, 40e of a rotatable disc 36, 40 (as shown in Figure 4), or adjacent the outer edges 37, 41 of an outer surface 36e, 40e of a rotatable disc 36, 40, or at the inner edges 39, 43 of an outer surface 36e, 40e of a rotatable disc 36, 40, or at any desired locations on the outer surface 36e, 40e of a rotatable disc 36, 40. A plurality of rows of teeth 52, 54 does not necessarily have to extend in a direction that is parallel to an edge of an outer surface 36e, 40e of a rotatable disc 36, 40 but may be extend at an angle to an edge of an outer surface 36e, 40e of a rotatable disc 36, 40. A plurality of rows of teeth 52, 54 may comprise or consist of adjacent rows of teeth, or may comprise or consist of spaced apart rows of teeth. The teeth of adjacent rows of teeth 52, 54 may arranged to be intermeshed or spaced apart from one another in the cross-machine direction.

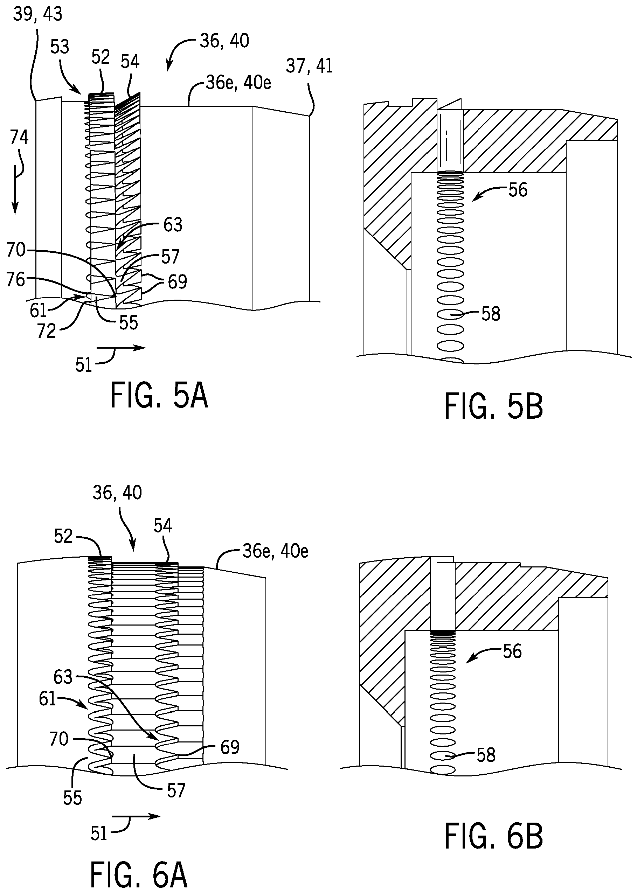

Figure 5A shows a rotatable disc 36, 40 having an outer surface 36e, 40e comprising two adjacent rows of teeth 52, 54 having pointed gripping ends 70, 69 and broad ends 61, 63. The rows of teeth 52, 54 protrude radially outwardly from the outer surface 36e, 40e. A groove 53 protrudes radially inward from the outer surface 36e, 40e and extends around the circumference of the rotatable disc 36, 40. The groove 53 is located between the inner edge 39, 43 of the rotatable disc 36, 40 and the first row of teeth 52. Each V-shaped tooth 55 in a first row of teeth 52 extends in an axial direction 51 of the rotatable disc 36, 40. Each of the V-shaped teeth 55 includes a pointed gripping end 70 that faces the axial direction 51, a first gripping edge 72 that generally faces the machine direction 74, and a second gripping edge 76 that generally faces 180 degrees opposite the first gripping edge 72 relative to the machine direction 74. The pointed gripping end 70 of teeth 55 may be positioned at the same radial distance from the outer surface 36e, 40e of the rotatable disc 36, 40 as second and third pointed gripping ends 72, 76 or at a greater radial distance according to alternate embodiments.

In the illustrated embodiment, each V-shaped tooth 57 in a second row of teeth 54 extends outward at an angle from the outer surface 36e, 40e of the rotatable disc 36, 40, thus defining V-shaped teeth 55 each having a pointed gripping end 69 that faces in the axial direction 51. These pointed gripping ends 69 thus aid in engaging and pulling the elastic strip 16 in the axial direction 51.

Depending on the direction of rotation of the rotatable disc 36, 40, either the first gripping edge 72 or the second gripping pointed edge 76 of V-shaped teeth 55 face in the machine direction 74 and thus aid in engaging and pulling the elastic strip 16 in the machine direction 74. Positioning groove 53 adjacent the V-shaped teeth 55 aids engagement between the elastic strip 16 and the first or second gripping edge 72, 76. The V-shaped teeth 55 thus engage the elastic strip 16 on at least two pointed gripping ends of the teeth 55 and thus facilitate pulling the elastic strip 16 in the axial direction 51 and machine direction 74, which aids in maintaining the elastic strip 16 in position on the rotatable disc 36, 40 in a stretched condition and also aids in encouraging the elastic strip 16 to rethread (i.e., re-stretch onto the rotatable disc 36, 40 in the axial direction 51) following a splice. The second row of teeth 54 provides additional pull on the elastic strip 16 in the axial direction 51 and may further aid in rethreading the elastic strip 16 following a splice. The second row of teeth 54 may be omitted in alternative embodiments.

According to an embodiment the teeth 55, 57 of a row of teeth 52, 54 may be either identical or non-identical as regards at least one or the following: their shape and/or their size (i.e. their length and/or their width and/or their height) and/or their direction of extension with respect to the axial direction of the rotatable disc 36, 40.

The outer surface 36e, 40e of each a rotatable disc 36, 40 comprises at least one aperture 58 that is configured to be fluidly connected to a vacuum pressure source (not shown). During operation, the vacuum pressure source draws air from the outside of the rotatable disc 36, 40 into the at least one aperture. The air then flows towards the vacuum pressure source. Figure 5B shows a row of apertures 56 located downstream of the plurality of rows of teeth 52, 54 through which air flows towards the vacuum pressure source. The vacuum pressure source causes the edge of an elastic strip 16 supported on the outer surface 36e, 40e of the rotatable disc 36, 40 to be pulled toward the at least one aperture 58 and between the rows of teeth 52, 54. The edge of the elastic strip 16 is thereby held on the outer surface 36e, 40e of the rotatable disc 36, 40 as the elastic strip 16 is advanced in the machine direction (MD) from the pick-up location to the drop-off location during operation.

Figure 6A shows a rotatable disc 36, 40 having an outer surface 36e, 40e comprising a plurality of rows of teeth 52, 54 including a narrow row of teeth 55 and a wide row of teeth 57 extending in an axial direction of the rotatable disc 36, 40. The tip end of each tooth 55, 57 is generally V-shaped and includes a respective pointed gripping end 69, 70 that faces the axial direction 51. Figure 6B shows the corresponding row of apertures 56 that is configured to be fluidly connected to a vacuum pressure source (not shown). The rows of teeth 52, 54 protrude radially outwardly from the outer surface 36, 40e of the rotatable disc 36, 40.

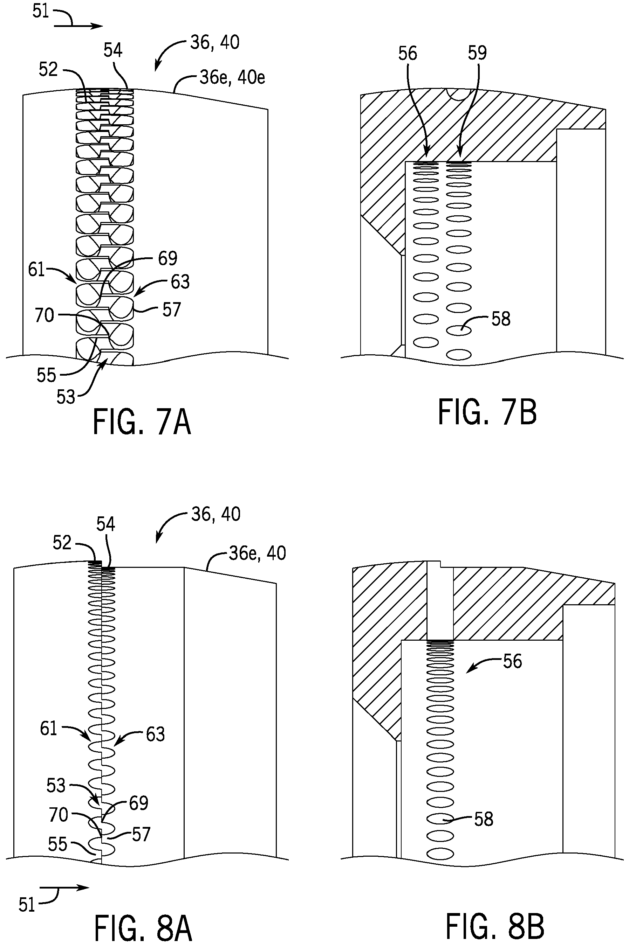

Figure 7A shows a rotatable disc 36, 40 having an outer surface 36e, 40e comprising two rows of bar-shaped teeth 52, 54, each of which includes a pointed gripping end 69, 70 that faces the axial direction 51. The rows of teeth 52, 54 do not protrude radially outwardly from the outer surface 36, 40e of the rotatable disc 36, 40 but lie flush with the outer surface 36e, 40e. Figure 7B shows a plurality of rows of apertures 56, 59 that is configured to be fluidly connected to a vacuum pressure source (not shown). The apertures 56, 59 are located within a recessed channel or groove 53 that follows a meandering path around the pointed gripping ends 69, 70 of teeth 52, 54. In operation, the vacuum pulled through aperture(s) 58 causes a portion of the elastic strip 16 to be drawn into the groove 53, where it is engaged by the pointed gripping ends 69, 70 of teeth 52, 54.

Any suitable aperture 58, 59, plurality of apertures 58, 59, arrangement or pattern of apertures 58, 59 may be used in an apparatus according to any embodiment of the present invention.

Figure 8A shows a rotatable disc 36, 40 having an outer surface 36e, 40e comprising two rows of teeth 52, 54. Each row of teeth comprises U-shaped teeth 55, 57 having a ridge. The rows of teeth 52, 54 do not protrude radially outwardly from the outer surface 36, 40e of the rotatable disc 36, 40 but lie flush with the outer surface 36e, 40e. Figure 8B shows a corresponding of row of apertures 56, 59 that is configured to be fluidly connected to a vacuum pressure source (not shown). The apertures 56, 59 are located within a recessed channel or groove 53 that follows a meandering path around the pointed gripping ends 69, 70 of teeth 52, 54.

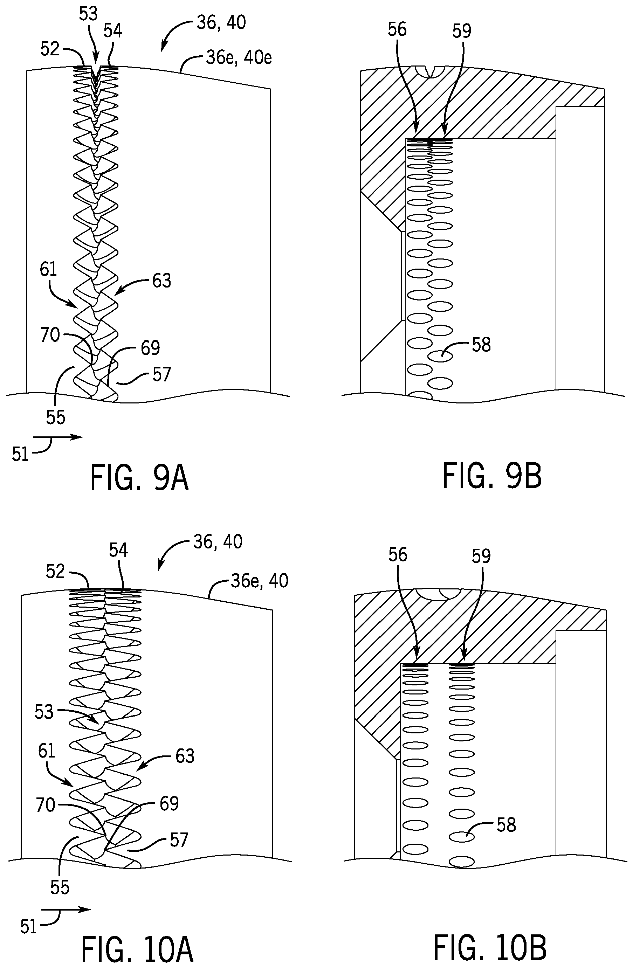

Figure 9A shows a rotatable disc 36, 40 having an outer surface 36e, 40e comprising two rows of V-shaped teeth 52, 54, each of which includes a pointed gripping end 69, 70 that face opposite one another in the axial direction 51, with a groove 53 between the rows of teeth 52, 54. The rows of teeth 52, 54 lie flush with the outer surface 36, 40e of the rotatable disc 36, 40. Figure 9B shows a corresponding plurality of rows of apertures 56, 59 that are configured to be fluidly connected to a vacuum pressure source (not shown).

Figure 10A shows a rotatable disc 36, 40 having an outer surface 36e, 40e comprising two rows of V-shaped teeth 52, 54 with a groove 53 between the rows of teeth 52, 54. The pointed gripping ends 69, 70 of V-shaped teeth 55, 57 are arranged to face in opposite directions along the axial direction 51 of the rotatable disc 36,40. The pointed gripping ends 69, 70 of the teeth 55 in row 52 face from left to right and the pointed gripping ends of the teeth 57 in row 54 face from right to left. The rows of teeth 52, 54 lie flush with the outer surface 36, 40e of the rotatable disc 36, 40. Figure 10B shows a corresponding plurality of rows of apertures 56, 59 that are configured to be fluidly connected to a vacuum pressure source (not shown).

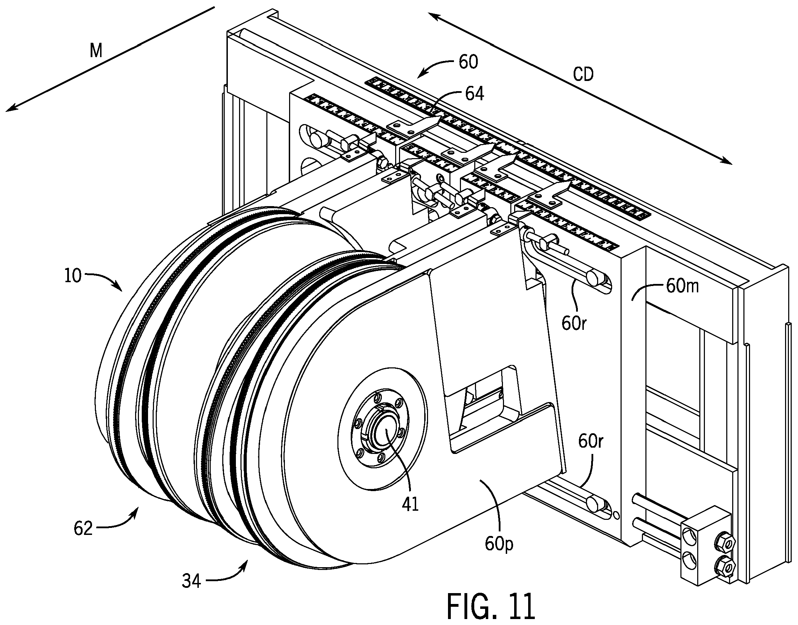

Figure 11 schematically shows a perspective view of a support structure 60 of an apparatus 10 according to an embodiment of the invention on which a first set of rotatable discs 34 is mounted at a distance from a second set of discs 62 in the cross-machine direction CD. It should be noted that a second set of discs 62 may be mounted in any desired location with respect to a first set of rotatable discs 34, such as upstream or downstream of the first set of rotatable discs 34 in the machine direction or omitted entirely. Additional sets of discs (not shown) may be included in alternative embodiments.

A support structure 60 may be configured to allow at least one rotatable disc 36, 40 to be moved in at least one of the following ways: along the machine direction MD, along a cross-machine direction CD, in a direction perpendicular to both machine direction MD and the cross-machine direction CD.

In the illustrated embodiment, each rotatable disc of a first set of rotatable discs 34 and a second set of rotatable discs 62 is slidably mounted on the support structure 60. Alternatively, one, some, or all of the rotatable discs 36, 40 of one or more sets of rotatable discs 34, 62 of an apparatus 10 may be mounted in a fixed, i.e. non-adjustable, position.

Each rotatable disc of each set of rotatable discs 34, 62 is connected to a side panel 60p the bottom and top ends of which are mounted on the rails 60r of a mounting plate 60m. The position and/or inclination of each rotatable disc of each set of rotatable discs 34, 62 can be adjusted by sliding the top and/or the bottom end of the side panel 60p along one or both of the rails 60r in the cross-machine direction CD.

At least one of the following parameters may be adjusted: the minimum distance 46 and/or the maximum distance 48 between the first rotatable disc 36 and the second rotatable disc 40 of a set of rotatable discs 34, a spacing between the first set of rotatable discs 34 and the second set or rotatable discs 62, whereby the spacing may be measured along the cross-machine direction CD between corresponding locations on each set of rotatable discs 34, 62, an inclination of a rotatable disc 36, 40, an inclination of an axis of rotation 38, 42. Once a desired parameter has been selected, each rotatable disc may be locked in place on the mounting structure 60.

The support structure 60 may comprise at least one rotatable disc-position indicators 64, in the form of at least one ruler and/or at least one protractor, configured to indicate a position and/or inclination of each rotatable disc that is mounted thereon.

Even though Figures 1-11 show rotatable discs having a circular shape and outer surface comprising at least one row of teeth that extends around the entire circumference of each rotatable disc, a rotatable disc does not necessarily have to have a circular shape or an outer surface comprising at least one row of teeth that extends arounds its entire circumference or perimeter.

A rotatable disc according to any of the embodiments of the invention may, for example, comprise an outer surface having one or more arms, nubs, or "shoes" that each extend radially outward from the rotatable disc. The one or more arms, nubs, or shoes may be mounted on the rotatable disc or integrally formed with the outer surface of the rotatable disc. An arm, nub, or shoe on a first rotatable disc of a set of rotatable discs may be aligned with an arm, nub, or shoe on a second rotatable disc of a set of rotatable discs to form a pair of arms, nubs or shoes that cooperate with each other to pick up an elastic strip from a vacuum drum. At least one aperture and at least one tooth of at least one row of teeth may be provided at the end of each arm, nub, or shoe to receive and grip the edge of an elastic strip and retain it in place using vacuum air pressure while the elastic strip is stretched in the cross-machine direction.

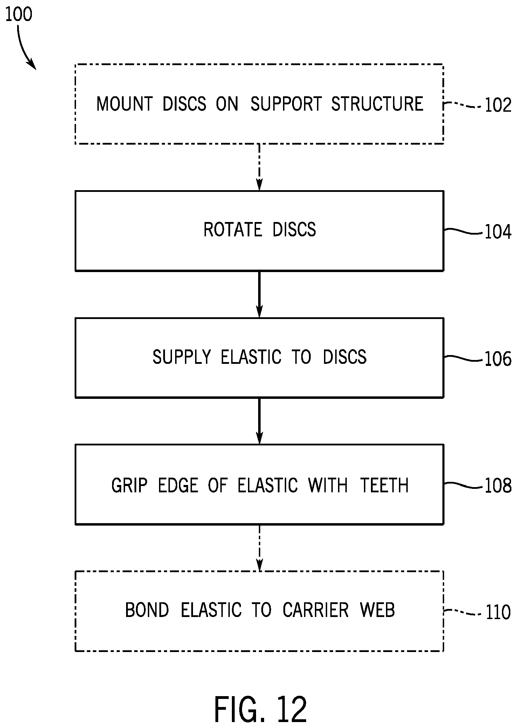

Figure 12 is a flow chart showing a method 100 according to an embodiment of the invention. Optional steps are shown using dashed lines.

The method comprises an optional step 102 of mounting at least one set of rotatable discs 34, 62, each having an outer surface comprising at least one aperture that is configured to be fluidly connected to a vacuum pressure source as described herein, on a support structure 60 and selecting a suitable arrangement of rotatable discs 36, 40. Preferably, at least one rotatable disc 36, 40 is slidably mounted on a support structure 60 that allows its position to be adjusted.

The method comprises a step 104 of rotating a first rotatable disc 36 and a second rotatable disc 40 of each set of rotatable discs 34, 62, whereby the first rotatable disc 36 is inclined at an angle with respect to the second rotatable disc 40.

The method comprises a step 106 of supplying an elastic strip to each set of rotatable discs 34, 62 for stretching each elastic strip 16 in a cross-machine direction CD.

The method further comprises a step 108 of receiving an edge of an elastic strip 16 on the outer surface 36e, 40e of the first rotatable disc 36 and an opposing edge of the elastic strip 16 on an outer surface 36e, 40e of the second rotatable disc 40 at a pick-up location, holding the elastic strip 16 on the outer surface 36e, 40e of each rotatable disc 36, 40 using the vacuum pressure source and advancing the elastic strip 16 in the machine direction MD from the pick-up location to a drop-off location, , whereby the distance between the first rotatable disc and the second rotatable disc is greater at the drop-off location than at the pick-up location, thereby stretching the elastic strip 16 in the cross-machine direction CD.

The outer surface 36e, 40e of each rotatable disc 36, 40 comprises at least one row of teeth 52, 54that extends around at least part of a periphery of a rotatable disc 36, 40, and that grips the elastic strip 16 that is pulled toward the at least one aperture 58 by the vacuum pressure source, thereby maintaining the hold on the edges of the elastic strip 16 as the elastic strip 16 is advanced in the machine direction MD from the pick-up location to the drop-off location during operation.

Optionally, the method comprises a step 110 of bonding the stretched elastic strip(s) 16 to at least one carrier web 24, 28 to produce an elastic laminate 12.

Preferences and options for a given aspect, feature or parameter of the invention should, unless the context indicates otherwise, be regarded as having been disclosed in combination with any and all preferences and options for all other aspects, features and parameters of the invention. For example, preferences and options disclosed in relation to an apparatus according to the present invention should be regarded as having been disclosed in combination with the method according to the present invention, and vice versa.

## Claims

1. An apparatus (10) for stretching an elastic strip (16) in a cross-machine direction (CD), the apparatus (10) comprising a set of rotatable discs (34, 62) including:
- a first rotatable disc (36), and
- a second rotatable disc (40),
whereby the first rotatable disc (36) is inclined at an angle with respect to the second rotatable disc (40), and whereby
each rotatable disc (36, 40) has an outer surface (36e, 40e) comprising at least one aperture (58) that is configured to be fluidly connected to a vacuum pressure source and configured to receive an edge of an elastic strip (16) at a pick-up location and hold the edge of the elastic strip (16) as the elastic strip (16) is advanced in the machine direction (MD) from the pick-up location to a drop-off location during operation, whereby the distance between the first rotatable disc (36) and the second rotatable disc (40) is greater at the drop-off location than at the pick-up location, thereby stretching the elastic strip (16) in the cross-machine direction (CD),
wherein the outer surface (36e, 40e) of each rotatable disc (36, 40) also comprises at least one row of teeth (52, 54) that extends around at least part of a periphery of a rotatable disc (36, 40), whereby the at least one row of teeth (52, 54) comprises a plurality of teeth (55, 57) each having a gripping end (69, 70), whereby each tooth of the plurality of teeth (55, 57) exhibits a maximum cross-section that is situated at a distance from at the gripping end (69, 70) of the tooth.

2. The apparatus (10) according to claim 1, wherein the outer surface (36e, 40e) comprises at least one groove (53) adjacent to the at least one row of teeth (52,54).

3. The apparatus (10) according to claim 1 or 2, wherein the at least one row of teeth (52, 54) lies flush with the outer surface (36e, 40e) of the rotatable disc (36, 40).

4. The apparatus (10) according to claim 1 or 2, wherein the at least one row of teeth (52, 54) protrudes radially outwards from the outer surface (36e, 40e) of the rotatable disc (36, 40).

5. The apparatus (10) according to any preceding claim, wherein the outer surface (36e, 40e) of at least one rotatable disc (36, 40) is integrally formed with the at least one row of teeth (52, 54).

6. The apparatus (10) according to any preceding claim, wherein at least one rotatable disc (36, 40) has an outer surface (36e, 40e) comprising a plurality of apertures (58), or at least at least one row of apertures (56), or a pattern of apertures (58), that extends around at least part of the periphery of a rotatable disc (36, 40).

7. The apparatus (10) according to any preceding claim, wherein the first rotatable disc (36) of is configured to rotate about a first axis of rotation (38), and the second rotatable disc (40) is configured to rotate about a second axis of rotation (42), whereby the first axis of rotation (38) is inclined at an angle with respect to the second axis of rotation (42)

8. The apparatus (10) according to any preceding claim, wherein the apparatus comprises at least one supply unit (14) configured to supply at least one elastic strip (16) to the set of rotatable discs (34, 62), and/or at least one supply unit (22, 26) configured to supply at least one carrier web (24, 28) to the set of rotatable discs (34, 62).

9. A method for stretching an elastic strip (16) in a cross-machine direction (CD), the method comprising:
- supplying an elastic strip (16) to a set of rotatable discs (34, 62) including a first rotatable disc (36) and a second rotatable disc (40), whereby each rotatable disc (36, 40) has an outer surface (36e, 40e) comprising at least one aperture (58) that is configured to be fluidly connected to a vacuum pressure source,
- rotating the first rotatable disc (36), and
- the second rotatable disc (40),
whereby the first rotatable disc (36) is inclined at an angle with respect to the second rotatable disc (40),
wherein the method comprises:
- receiving an edge of the elastic strip (16) on the outer surface (36e, 40e) of the first rotatable disc (36) and an opposing edge of the elastic strip (16) on the outer surface (36e, 40e) of the second rotatable disc (40) at a pick-up location, wherein the outer surface (36e, 40e) of each rotatable disc (36, 40) comprises at least one row of teeth (52, 54) that extends around at least part of a periphery of a rotatable disc (36,40), and that comprises a plurality of teeth (55, 57) each having a plurality of teeth having a gripping end (69, 70), whereby each tooth of the plurality of teeth (55, 57) exhibits a maximum cross-section that is situated at a distance from at the gripping end (69, 70) of the tooth,
- holding the elastic strip (16) on the outer surface (36e, 40e) of each rotatable disc (36, 40) using the vacuum pressure source, and
- advancing the elastic strip (16) in the machine direction (MD) from the pick-up location to a drop-off location, whereby the distance between the first rotatable disc (36) and the second rotatable disc (40) is greater at the drop-off location than at the pick-up location, thereby stretching the elastic strip (16) in the cross-machine direction (CD).

10. The method according to claim 9, wherein the outer surface (36e, 40e) of each rotatable disc (36, 40) of a set of rotatable discs (34, 62) comprises at least one row of teeth (52, 54) that lies flush with the outer surface (36e, 40e) of the rotatable disc (36, 40).

11. The method according to claim 9, wherein the outer surface (36e, 40e) of each rotatable disc (36, 40) of a set of rotatable discs (34, 62) comprises at least one row of teeth (52, 54) that protrudes radially outwards from the outer surface (36e, 40e) of the rotatable disc (36, 40).

12. The method according to any of claims 9-11, wherein the at least one rotatable disc (36, 40) comprises a plurality of apertures (58), or at least one row of apertures (56), or a pattern of apertures (58) that extends around at least part of the periphery of the at least one rotatable disc (36, 40).

13. The method according to any of claims 9-12, wherein at least one rotatable disc (36, 40) comprises an outer surface (36e, 40e) that is integrally formed with the at least one row of teeth (52, 54).

14. The method according to any of claims 9-13, wherein the method comprises:
- supplying at least one carrier web (24, 28), and
- bonding the stretched elastic strip (16) to the at least one carrier web (24, 28).

15. Use of an apparatus (10) according to any of claims 1-8 or a method according to any of claims 9-14 to assemble an elastic laminate (12) and/or to manufacture an absorbent article.

## Patentansprüche

1. Einrichtung (10) zum Dehnen eines elastischen Streifens (16) in einer Maschinenquerrichtung (CD), wobei die Einrichtung (10) einen Satz drehbarer Scheiben (34, 62) umfasst, einschließlich:
- einer ersten drehbaren Scheibe (36), und
- einer zweiten drehbaren Scheibe (40),
wobei die erste drehbare Scheibe (36) in einem Winkel in Bezug auf die zweite drehbare Scheibe (40) geneigt ist, und wobei
jede drehbare Scheibe (36, 40) eine Außenoberfläche (36e, 40e) aufweist, die mindestens eine Öffnung (58) umfasst, die konfiguriert ist, um fluidisch mit einer Unterdruckquelle verbunden zu werden, und konfiguriert ist, um eine Kante eines elastischen Streifens (16) an einer Aufnahmestelle aufzunehmen und die Kante des elastischen Streifens (16) zu halten, während der elastische Streifen (16) während des Betriebs in der Maschinenrichtung (MD) von der Aufnahmestelle zu einer Abgabestelle vorbewegt wird, wobei der Abstand zwischen der ersten drehbaren Scheibe (36) und der zweiten drehbaren Scheibe (40) an der Abgabestelle größer ist als an der Aufnahmestelle, wodurch der elastische Streifen (16) in der Maschinenquerrichtung (CD) gedehnt wird,
wobei die Außenoberfläche (36e, 40e) jeder drehbaren Scheibe (36, 40) ebenfalls mindestens eine Zahnreihe (52, 54) umfasst, die sich um mindestens einen Teil eines Umfangs einer drehbaren Scheibe (36, 40) erstreckt, wobei die mindestens eine Zahnreihe (52, 54) eine Vielzahl von Zähnen (55, 57) umfasst, die jeweils ein Greifende (69, 70) aufweisen, wobei jeder Zahn der Vielzahl von Zähnen (55, 57) einen maximalen Querschnitt vorweist, der sich in einem Abstand von dem Greifende (69, 70) des Zahns befindet.

2. Einrichtung (10) nach Anspruch 1, wobei die Außenoberfläche (36e, 40e) mindestens eine Nut (53) benachbart zu der mindestens einen Zahnreihe (52, 54) umfasst.

3. Einrichtung (10) nach Anspruch 1 oder 2, wobei die mindestens eine Zahnreihe (52, 54) bündig mit der Außenoberfläche (36e, 40e) der drehbaren Scheibe (36, 40) abschließt.

4. Einrichtung (10) nach Anspruch 1 oder 2, wobei die mindestens eine Zahnreihe (52, 54) von der Außenoberfläche (36e, 40e) der drehbaren Scheibe (36, 40) radial nach außen vorsteht.

5. Einrichtung (10) nach einem der vorstehenden Ansprüche, wobei die Außenoberfläche (36e, 40e) mindestens einer drehbaren Scheibe (36, 40) einstückig mit der mindestens einen Zahnreihe (52, 54) ausgebildet ist.

6. Einrichtung (10) nach einem der vorstehenden Ansprüche, wobei mindestens eine drehbare Scheibe (36, 40) eine Außenoberfläche (36e, 40e) aufweist, die eine Vielzahl von Öffnungen (58) oder mindestens eine Reihe von Öffnungen (56) oder ein Muster von Öffnungen (58) umfasst, die sich um mindestens einen Teil des Umfangs einer drehbaren Scheibe (36, 40) erstrecken.

7. Einrichtung (10) nach einem der vorstehenden Ansprüche, wobei die erste drehbare Scheibe (36) konfiguriert ist, um sich um eine erste Drehachse (38) zu drehen, und die zweite drehbare Scheibe (40) konfiguriert ist, um sich um eine zweite Drehachse (42) zu drehen, wobei die erste Drehachse (38) in einem Winkel in Bezug auf die zweite Drehachse (42) geneigt ist.

8. Einrichtung (10) nach einem der vorstehenden Ansprüche, wobei die Einrichtung mindestens eine Versorgungseinheit (14), die konfiguriert ist, um dem Satz drehbarer Scheiben (34, 62) mindestens einen elastischen Streifen (16) zuzuführen, und/oder mindestens eine Versorgungseinheit (22, 26), die konfiguriert ist, um dem Satz drehbarer Scheiben (34, 62) mindestens eine Trägerbahn (24, 28) zuzuführen, umfasst.

9. Verfahren zum Dehnen eines elastischen Streifens (16) in einer Maschinenquerrichtung (CD), das Verfahren umfassend:
- Zuführen eines elastischen Streifens (16) zu einem Satz drehbarer Scheiben (34, 62) einschließlich einer ersten drehbaren Scheibe (36) und einer zweiten drehbaren Scheibe (40), wobei jede drehbare Scheibe (36, 40) eine Außenoberfläche (36e, 40e) aufweist, die mindestens eine Öffnung (58) umfasst, die konfiguriert ist, um fluidisch mit einer Vakuumdruckquelle verbunden zu werden,
- Drehen der ersten drehbaren Scheibe (36), und
- der zweiten drehbaren Scheibe (40),
wobei die erste drehbare Scheibe (36) in einem Winkel in Bezug auf die zweite drehbare Scheibe (40) geneigt ist,
wobei das Verfahren umfasst:
- Aufnehmen einer Kante des elastischen Streifens (16) auf der Außenoberfläche (36e, 40e) der ersten drehbaren Scheibe (36) und einer gegenüberliegenden Kante des elastischen Streifens (16) auf der Außenoberfläche (36e, 40e) der zweiten drehbaren Scheibe (40) an einer Aufnahmestelle, wobei die Außenoberfläche (36e, 40e) jeder drehbaren Scheibe (36, 40) mindestens eine Zahnreihe (52, 54) umfasst, die sich um mindestens einen Teil eines Umfangs einer drehbaren Scheibe (36, 40) erstreckt und die eine Vielzahl von Zähnen (55, 57) umfasst, die jeweils eine Vielzahl von Zähnen mit einem Greifende (69, 70) aufweisen, wobei jeder Zahn der Vielzahl von Zähnen (55, 57) einen maximalen Querschnitt aufweist, der sich in einem Abstand von dem Greifende (69, 70) des Zahns befindet,
- Halten des elastischen Streifens (16) an der Außenoberfläche (36e, 40e) jeder drehbaren Scheibe (36, 40) mittels der Vakuumdruckquelle, und
- Vorbewegen des elastischen Streifens (16) in der Maschinenrichtung (MD) von der Aufnahmestelle zu einer Abgabestelle, wobei der Abstand zwischen der ersten drehbaren Scheibe (36) und der zweiten drehbaren Scheibe (40) an der Abgabestelle größer ist als an der Aufnahmestelle, wodurch der elastische Streifen (16) in der Maschinenquerrichtung (CD) gedehnt wird.

10. Verfahren nach Anspruch 9, wobei die Außenoberfläche (36e, 40e) jeder drehbaren Scheibe (36, 40) eines Satzes von drehbaren Scheiben (34, 62) mindestens eine Zahnreihe (52, 54) umfasst, die bündig mit der Außenoberfläche (36e, 40e) der drehbaren Scheibe (36, 40) liegt.

11. Verfahren nach Anspruch 9, wobei die Außenoberfläche (36e, 40e) jeder drehbaren Scheibe (36, 40) eines Satzes von drehbaren Scheiben (34, 62) mindestens eine Zahnreihe (52, 54) umfasst, die von der Außenoberfläche (36e, 40e) der drehbaren Scheibe (36, 40) radial nach außen vorsteht.

12. Verfahren nach einem der Ansprüche 9 bis 11, wobei die mindestens eine drehbare Scheibe (36, 40) eine Vielzahl von Öffnungen (58) oder mindestens eine Reihe von Öffnungen (56) oder ein Muster von Öffnungen (58) umfasst, das sich um mindestens einen Teil des Umfangs der mindestens einen drehbaren Scheibe (36, 40) erstreckt.

13. Verfahren nach einem der Ansprüche 9 bis 12, wobei mindestens eine drehbare Scheibe (36, 40) eine Außenoberfläche (36e, 40e) umfasst, die einstückig mit der mindestens einen Zahnreihe (52, 54) ausgebildet ist.

14. Verfahren nach einem der Ansprüche 9 bis 13, wobei das Verfahren umfasst:
- Bereitstellen mindestens einer Trägerbahn (24, 28), und
- Verbinden des gedehnten elastischen Streifens (16) mit der mindestens einen Trägerbahn (24, 28).

15. Verwendung einer Einrichtung (10) nach einem der Ansprüche 1 bis 8 oder eines Verfahrens nach einem der Ansprüche 9 bis 14 zum Zusammensetzen eines elastischen Laminats (12) und/oder zum Herstellen eines absorbierenden Artikels.

## Revendications

1. Appareil (10) permettant d'étirer une bande élastique (16) dans un sens transversal de la machine (CD), l'appareil (10) comprenant un ensemble de disques rotatifs (34, 62) comportant :
- un premier disque rotatif (36), et
- un second disque rotatif (40),
moyennant quoi le premier disque rotatif (36) est incliné à un angle par rapport au second disque rotatif (40), et moyennant quoi
chaque disque rotatif (36, 40) a une surface externe (36e, 40e) comprenant au moins une ouverture (58) qui est conçue pour être reliée fluidiquement à une source de pression sous vide et conçue pour recevoir un bord d'une bande élastique (16) au niveau d'un emplacement de collecte et maintenir le bord de la bande élastique (16) lorsque la bande élastique (16) est avancée dans le sens machine (MD) de l'emplacement de collecte à un emplacement de dépôt pendant le fonctionnement, moyennant quoi la distance entre le premier disque rotatif (36) et le second disque rotatif (40) est plus grande au niveau de l'emplacement de dépôt qu'au niveau de l'emplacement de collecte, étirant de ce fait la bande élastique (16) dans le sens transversal de la machine (CD),
dans lequel la surface externe (36e, 40e) de chaque disque rotatif (36, 40) comprend également au moins une rangée de dents (52, 54) qui s'étend autour d'au moins une partie d'une périphérie d'un disque rotatif (36, 40), moyennant quoi l'au moins une rangée de dents (52, 54) comprend une pluralité de dents (55, 57) ayant chacune une extrémité de préhension (69, 70), moyennant quoi chaque dent de la pluralité de dents (55, 57) présente une section transversale maximale qui est située à une distance du niveau de l'extrémité de préhension (69, 70) de la dent.

2. Appareil (10) selon la revendication 1, dans lequel la surface externe (36e, 40e) comprend au moins une rainure (53) adjacente à l'au moins une rangée de dents (52, 54).

3. Appareil (10) selon la revendication 1 ou 2, dans lequel l'au moins une rangée de dents (52, 54) affleure la surface externe (36e, 40e) du disque rotatif (36, 40).

4. Appareil (10) selon la revendication 1 ou 2, dans lequel l'au moins une rangée de dents (52, 54) fait saillie radialement vers l'extérieur à partir de la surface externe (36e, 40e) du disque rotatif (36, 40).

5. Appareil (10) selon l'une quelconque revendication précédente, dans lequel la surface externe (36e, 40e) d'au moins un disque rotatif (36, 40) est formée intégralement avec l'au moins une rangée de dents (52, 54).

6. Appareil (10) selon l'une quelconque revendication précédente, dans lequel au moins un disque rotatif (36, 40) a une surface externe (36e, 40e) comprenant une pluralité d'ouvertures (58), ou au moins une rangée d'ouvertures (56), ou un motif d'ouvertures (58), qui s'étend autour d'au moins une partie de la périphérie d'un disque rotatif (36, 40).

7. Appareil (10) selon l'une quelconque revendication précédente, dans lequel le premier disque rotatif (36) est conçu pour tourner autour d'un premier axe de rotation (38), et le second disque rotatif (40) est conçu pour tourner autour d'un second axe de rotation (42), moyennant quoi le premier axe de rotation (38) est incliné à un angle par rapport au second axe de rotation (42)

8. Appareil (10) selon l'une quelconque revendication précédente, dans lequel l'appareil comprend au moins une unité d'alimentation (14) conçue pour fournir au moins une bande élastique (16) à l'ensemble de disques rotatifs (34, 62), et/ou au moins une unité d'alimentation (22, 26) conçue pour fournir au moins une bande de support (24, 28) à l'ensemble de disques rotatifs (34, 62).

9. Procédé permettant d'étirer une bande élastique (16) dans un sens transversal de la machine (CD), le procédé comprenant :
- la fourniture d'une bande élastique (16) à un ensemble de disques rotatifs (34, 62) comportant un premier disque rotatif (36) et un second disque rotatif (40), moyennant quoi chaque disque rotatif (36, 40) a une surface externe (36e, 40e) comprenant au moins une ouverture (58) qui est conçue pour être reliée fluidiquement à une source de pression sous vide,
- la rotation du premier disque rotatif (36), et
- le second disque rotatif (40),
moyennant quoi le premier disque rotatif (36) est incliné à un angle par rapport au second disque rotatif (40),
le procédé comprenant :
- la réception d'un bord de la bande élastique (16) sur la surface externe (36e, 40e) du premier disque rotatif (36) et d'un bord opposé de la bande élastique (16) sur la surface externe (36e, 40e) du second disque rotatif (40) au niveau d'un emplacement de collecte, la surface externe (36e, 40e) de chaque disque rotatif (36, 40) comprenant au moins une rangée de dents (52, 54) qui s'étend autour d'au moins une partie d'une périphérie d'un disque rotatif (36, 40), et qui comprend une pluralité de dents (55, 57) ayant chacune une pluralité de dents ayant une extrémité de préhension (69, 70), moyennant quoi chaque dent de la pluralité de dents (55, 57) présente une section transversale maximale qui est située à une distance du niveau de l'extrémité de préhension (69, 70) de la dent,
- le maintien de la bande élastique (16) sur la surface externe (36e, 40e) de chaque disque rotatif (36, 40) à l'aide de la source de pression sous vide, et
- l'avancement de la bande élastique (16) dans le sens machine (MD) de l'emplacement de collecte à un emplacement de dépôt, moyennant quoi la distance entre le premier disque rotatif (36) et le second disque rotatif (40) est plus grande au niveau de l'emplacement de dépôt qu'au niveau de l'emplacement de collecte, étirant de ce fait la bande élastique (16) dans le sens transversal de la machine (CD).

10. Procédé selon la revendication 9, dans lequel la surface externe (36e, 40e) de chaque disque rotatif (36, 40) d'un ensemble de disques rotatifs (34, 62) comprend au moins une rangée de dents (52, 54) qui affleure la surface externe (36e, 40e) du disque rotatif (36, 40).

11. Procédé selon la revendication 9, dans lequel la surface externe (36e, 40e) de chaque disque rotatif (36, 40) d'un ensemble de disques rotatifs (34, 62) comprend au moins une rangée de dents (52, 54) qui fait saillie radialement vers l'extérieur à partir de la surface externe (36e, 40e) du disque rotatif (36, 40).

12. Procédé selon l'une quelconque des revendications 9 à 11, dans lequel l'au moins un disque rotatif (36, 40) comprend une pluralité d'ouvertures (58), ou au moins une rangée d'ouvertures (56), ou un motif d'ouvertures (58) qui s'étend autour d'au moins une partie de la périphérie de l'au moins un disque rotatif (36, 40).

13. Procédé selon l'une quelconque des revendications 9 à 12, dans lequel au moins un disque rotatif (36, 40) comprend une surface externe (36e, 40e) qui est formée d'un seul tenant avec l'au moins une rangée de dents (52, 54).

14. Procédé selon l'une quelconque des revendications 9 à 13, dans lequel le procédé comprend :
- la fourniture d'au moins une bande de support (24, 28), et
- la liaison de la bande élastique étirée (16) à l'au moins une bande de support (24, 28).

15. Utilisation d'un appareil (10) selon l'une quelconque des revendications 1 à 8 ou procédé selon l'une quelconque des revendications 9 à 14 pour assembler un stratifié élastique (12) et/ou pour fabriquer un article absorbant.
